# DEMANDE DE BREVET EUROPEEN

(11) **EP 1 393 736 A1**
(43) Date de publication de la demande: **03.03.2004**
(21) Numéro de dépôt: 03078241.1
(22) Date de dépôt: 24.10.1997
(51) Int. Cl.: A61K 35/78

(54) **Utilisation d'un extrait flavonoidique de Ginkgo biloba substantiellement dépourvu de terpènes, dans le domaine bucco-dentaire, et composition contenant un tel extrait**

(30) Priorité: 25.10.1996 FR 9613065
(62) Demande divisionnaire de: 97912251.2
(71) Demandeur: Société de Conseils de Recherches et d'Applications Scientifiques ( S.C.R.A.S.), 75016 Paris (FR)
(72) Inventeur: O'Reilly, Joseph, Glounthaune County Cork (IE)
(74) Mandataire: Bourgouin, André

(57) **Abrégé**

L'invention concerne l'utilisation d'un extrait flavonoïdique de feuilles de ginkgo biloba, et plus spécifiquement un extrait dépourvu de terpènes dans le domaine bucco-dentaire. L'invention concerne également une composition bucco-dentaire contenant un tel extrait.

## Description

L'invention concerne l'utilisation d'un extrait flavonoïdique de ginkgo biloba, et plus spécifiquement d'un extrait substantiellement dépourvu de terpènes, dans le domaine bucco-dentaire. L'invention concerne également une composition bucco-dentaire contenant un tel extrait.

Un extrait flavonoïdique de feuilles de ginkgo biloba dépourvu de terpènes selon l'invention, comprend des hétérosides flavonoïdiques et peu ou pas de terpènes. Lorsque l'extrait contient des terpènes, le taux de terpènes est de 1 % au maximum, et de préférence de 0,5 % au maximum. Cet extrait contient de 28 à 35 % d'hétérosides flavonoïdiques, et de préférence 28 à 32 %. De tels extraits sont obtenus de préférence à partir de feuilles de jeunes arbres de ginkgo biloba taillés.

L'invention a pour objet un procédé d'obtention d'un tel extrait, procédé qui comprend plusieurs étapes d'extraction d'extrait de feuilles de ginkgo biloba par des solvants et caractérisé en ce qu'une des étapes d'extraction est une étape de déterpénation et le solvant utilisé est un composé de formule RC(O)OR' dans laquelle R et R' représentent, indépendamment, un alkyl inférieur, seul ou mélangé avec un hydrocarbure aliphatique saturé contenant au moins 5 atomes de carbone. L'étape d'extraction peut être effectuée à n'importe quel stade du procédé. De préférence, le solvant utilisé lors de l'étape de déterpénation, comprend de 0 à 20 % d'hydrocarbure aliphatique saturé.

Des étapes d'extraction autres que l'étape de déterpénation, sont connues dans la littérature notamment dans les brevets EP431535, EP 431536, EP 360556 et EP324197. Ces brevets sont incorporés par référence dans la présente demande.

Dans les définitions indiquées ci-dessus, l'expression alkyle inférieure représente de préférence un radical alkyle ayant de 1 à 6 atomes de carbone linéaire ou ramifié et en particulier un radical alkyle ayant de 1 à 4 atomes de carbone tels que les radicaux méthyle, éthyle, propyle, isopropyle, butyle, isobutyle, sec-butyle et tert-butyle. Les solvants de formule RC(O)OR' dans laquelle les radicaux R et R' représentent méthyle, éthyle ou propyle, et notamment l'acétate d'éthyle, sont préférentiellement utilisés. L'hydrocarbure aliphatique saturé peut être choisi parmi l'hexane, l'heptane, l'octane. De préférence, l'heptane est utilisé.

Un extrait flavonoïdique de feuilles de ginkgo biloba tel que défini ci-dessus présente une activité anti-élastasique et anti-inflammatoire sur des gencives humaines. On trouvera ci-après, dans la partie expérimentale, une illustration de ces propriétés.

Ces propriétés rendent l'extrait de ginkgo tel que défini ci-dessus apte à une utilisation pharmaceutique. La présente demande a donc pour objet, à titre de médicament, et notamment à titre de médicament destiné à la sphère buccodentaire, un extrait flavonoïdique de feuilles de ginkgo biloba tel que défini ci-dessus.

L'invention a également pour objet une composition pharmaceutique destinée notamment au traitement de la sphère buccodentaire et contenant comme principe actif un extrait flavonoïdique de feuilles de ginkgo biloba tel que défini ci-dessus, éventuellement en association avec un support pharmaceutiquement acceptable.

L'invention a plus particulièrement pour objet une composition pharmaceutique anti-élastasique contenant, à titre d'agent anti-élastasique, au moins une quantité efficace d'un extrait de ginkgo tel que défini ci-dessus. L'invention a plus particulièrement pour objet également une composition pharmaceutique anti-inflammatoire contenant, à titre d'agent anti-inflammatoire, au moins une quantité efficace d'un extrait de ginkgo tel que défini ci-dessus.

De préférence, une composition pharmaceutique selon l'invention comprend de 0,05 à 0,6 % d'extrait de ginkgo, et de manière préférencielle de 0,1 à 0,5 %. Une telle composition peut contenir également au moins un autre agent ayant des activités similaires ou complémentaires. De préférence, une composition pharmaceutique selon l'invention contient de l'extrait de ginkgo tel que défini ci-dessus en association avec des céramides. De manière préférentielle, une telle composition comprend de 0,1 à 0,5 % d'extrait de ginkgo et de 0,05 à 0,3 % de céramides.

Une composition pharmaceutique selon l'invention peut se présenter sous toute forme appropriée selon le mode d'administration choisie. Elle peut se présenter sous forme liquide comme, par exemple, une solution, une suspension, une émulsion. Elle peut également se présenter sous forme de gel, de spray, de pâte dentifrice, de chewing-gum. De préférence, elle se présente sous forme de gel ou de chewing-gum. Elle peut contenir, outre l'extrait de ginkgo, seul ou associé, et un support approprié, les ingrédients ou adjuvants habituellement utilisés pour la réalisation de telles compositions.

L'invention a également pour objet l'utilisation d'un extrait flavonoïdique de ginkgo tel que défini ci-dessus, pour la préparation d'une composition pharmaceutique destinée au traitement de la sphère bucco-dentaire.

L'invention a plus particulièrement pour objet l'utilisation d'un extrait flavonoïdique de ginkgo tel que défini ci-dessus, pour la préparation d'une composition pharmaceutique destinée à inhiber l'élastase. L'invention a plus particulièrement pour objet également l'utilisation d'un extrait flavonoïdique de ginkgo tel que défini ci-dessus, pour la préparation d'une composition pharmaceutique destinée à inhiber les inflammations de la sphère buccodentaire.Un tel extrait flavonoïdique peut être utilisé seul ou en association avec un autre agent ayant des propriétés similaires ou complémentaires suivant le résultat recherché, éventuellement en présence d'un support approprié. De préférence, un extrait tel que défini ci-dessus est utilisé en association avec des céramides.

Les céramides sont des sphingolipides présentes dans toutes les structures cellulaires. Elles sont également très présentes dans le monde végétale et en particulier dans le blé, le riz, le soja, le millet et les épinards. Elles peuvent également être synthétisées. En raison de leur structure, elles peuvent avantageusement être utilisées en tant que vecteur, en augmentant considérablement la biodisponibilité de l'agent actif qu'elles véhiculent. Le terme céramide utilisé dans la présente demande a la signification classique connue de l'homme de l'art. Ainsi le terme céramide comprend toutes les céramides, d'origine synthétique ou naturelle (végétale, animale ou humaine) éventuellement substituées, par exemple, par un sucre telles que les mono ou polyglucosylcéramides.

En raison d'un taux élevé en hétérosides flavonoïdiques, l'état de la technique suggère qu'un extrait tel que défini ci-dessus, présente une activité anti-oxydante et anti-radicalaire. Par ailleurs, compte tenu, d'une part de ses propriétés anti-inflammatoire, anti-élastasique mais également vasorégulatrice et activatrice de collagène et, d'autre part, d'une absence presque totale de terpènes dans sa composition, un tel extrait peut avantageusement être utilisé en cosmétique ou en dermatologie.

L'invention a également pour objet l'utilisation d'un extrait flavonoïdique de feuilles de ginkgo biloba tel que défini ci-dessus, pour la préparation d'un médicament vasorégulateur et/ou activateur de collagène. L'invention concerne également l'utilisation d'une composition cosmétique comprenant un extrait flavonoïdique tel que défini ci-dessus comme vasorégulateur et/ou activateur de collagène. L'invention a également pour objet une méthode de traitement cosmétique caractérisée en ce que l'on utilise un extrait flavonoïdique tel que défini ci-dessus comme vasorégulateur et/ou activateur de collagène.

L'invention a également pour objet une composition cosmétique destinée notamment à la sphère buccodentaire et contenant un extrait flavonoïdique de feuilles de ginkgo biloba tel que défini ci-dessus. Cet extrait peut être utilisé seul ou en association avec au moins un autre agent ayant une activité similaire ou complémentaire, et éventuellement avec un support approprié. De préférence, l'extrait de ginkgo est utilisé en association avec des céramides. Une composition cosmétique selon l'invention peut contenir de 0,05 à 0,6 % d'extrait de ginkgo tel que défini ci-dessus, et de 0,05 à 0,3 % de céramides. Une telle composition peut se présenter sous toute forme appropriée dans ce type d'application telle que crèmes, émulsions, laits, gels, huiles, produits de maquillage, bâtons de rouge à lèvre, lotions. Elle peut contenir, outre l'extrait de ginkgo, seul ou associé, et un support approprié, les ingrédients ou adjuvants habituellement utilisés pour la réalisation de telles compositions.

L'invention a également pour objet l'utilisation d'un extrait flavonoïdique de ginkgo biloba tel que défini ci-dessus pour le traitement cosmétique des affections de la sphère buccodentaire et notamment de la gingivite.

Les exemples suivants sont présentés pour illustrer l'invention et ne doivent en aucun cas être considérés comme une limite à la portée de l'invention.

### Partie expérimentale

### Procédé d'obtention d'un extrait flavonoïdique

Les feuilles de ginkgo biloba sont extraites avec de 6 à 12 parties (de préférence 8) d'eau contenant 60 % d'acétone à 50-60° C et la solution est concentrée de manière à réduire le pourcentage en acétone à moins de 3 %. Cette solution est refroidie et les lipides sont éliminés par décantation. La solution aqueuse est extraite avec de 2 à 5 parties d'acétate d'éthyl contenant de 0 à 20 % d'heptane. La solution résultante est extraite avec une quantité minimum d'un mélange acétone / butanol (pourcentage d'acétone 0 à 15 %) en présence de sulfate d'ammonium. La phase organique est concentrée ; après ajout d'éthanol, la solution est concentrée de nouveau. Après une nouvelle dilution à l'éthanol, la solution est refroidie et des précipités insolubles sont filtrés. La solution résultante est concentrée, séchée et enfin broyée pour récupérer l'extrait flavonoïdique sous forme d'une poudre homogène.

### Etude de l'activité anti-élastasique et anti-inflammatoire d'un extrait flavonoïdique de ginkgo biloba substantiellement dépourvu de terpène

### 1- Etude de l'activité anti-élastasique ex vivo

Le but de cette étude est donc d'étudier l'activité anti-élastasique d'un extrait tel que défini ci-dessus, seul ou en association avec des céramides. Cette activité est évaluée sur des coupes de gencives humaines.

Les céramides utilisées, d'origine végétale, sont obtenues selon un procédé tel que décrit dans le brevet FR 2676936 incorporé par référence, procédé qui comprend l'extraction de blé, en utilisant un solvant d'extraction polaire suivie d'une recristallisation dans un solvant organique. Elles se composent essentiellement de glucosylcéramides et de céramides.

Pour cela, le réseau de fibres élastiques est soumis à l'élastase leucocytaire humaine (ELH), avec ou sans produit à visée anti-élastasique. Les fibres élastiques qui subsistent après une action enzymatique sont colorées par la (+) catéchine.

Des coupes congelées de 8 µm sont réalisées à partir de 5 prélèvements obtenus à partir de biopsies de muqueuse buccale normale, prélevées lors de gestes d'avulsion dentaire.

### Protocole:

Dans un premier temps, la concentration en élastase leucocytaire humaine nécessaire à la destruction des fibres élastiques, est déterminée : l'élastase leucocytaire est appliquée sur des coupes de peau pendant deux heures à température ambiante et en atmosphère humide ; après fixation dans l'acétone et déshydratation dans de l'éthanol à 70 °C, les fibres élastiques restantes sont colorées par la (+) catéchine. La destruction totale des fibres élastiques est obtenue en présence de 10 µm/ml d'élastase leucocytaire humaine.

Afin de montrer une éventuelle fixation des produits sur l'enzyme, des quantités similaires de produits, sous forme d'émulsion, et d'élastase leucocytaire humaine à 10 µm/ml sont appliquées simultanément sur les coupes pendant deux heures, à température ambiante. L'activité anti-élastasique des produits est comparée à celle du (PMSF), inhibiteur puissant des élastases.

La quantification morphométrique - par analyse d'images - donne les résultats qui sont rassemblés dans le tableau 1 ci dessous.

**Tableau 1:**

| % de fibres élastiques restantes après traitement par l'élastase leucocytaire humaine en présence d'extrait de ginkgo seul ou en association avec des céramides | | | | |
|---|---|---|---|---|
| | | chorion supérieur | chorion moyen | chorion inférieur |
| ELH seule | | 0 | 0 | 0 |
| ELH + PMSF | | 25 | 55 | 70 |
| Extrait de ginkgo + | Céramide | | | |
| (en %) | (en %) | | | |
| 0 | 0,1 | 0 | 0 | 27,65 |
| 0 | 0,2 | 0 | 0 | 22,50 |
| 0,1 | 0 | 0 | 0 | 13,85 |
| 0,3 | 0 | 0 | 0 | 33 |
| 0,5 | 0 | 2,5 | 31,85 | 61,20 |
| 0,1 | 0,1 | 0 | 0 | 24,5 |
| 0,3 | 0,1 | 0 | 17,6 | 23 |
| 0,5 | 0,1 | 56,7 | 47 | 65 |
| 0,1 | 0,2 | 0 | 0 | 15,2 |
| 0,3 | 0,2 | 0 | 19 | 30 |
| 0,5 | 0,2 | 70 | 53,2 | 80 |

### 2- Etude de l'activité anti-inflammatoire

La composition testée dans cette étude, se présente sous forme de gel et comprend 0,5 % d'extrait de ginkgo tel que défini ci-dessus et 0,1 % de céramide végétale telle que définie dans l'étude de l'activité antiélastasique.

L'étude est réalisée sur 20 patients atteints d'une gengivite tartrique bénigne et modérée. Les patients appliquent le gel deux fois par jour, pendant un mois, au niveau de leur muqueuse gingivale. L'appréciation clinique de l'état gingival est réalisée aux jours J₀ et J₂₈ à l'aide d'un système de cotation. Les critères principaux d'évaluation sont l'appréciation de l'érythème et de la gingivite. L'action anti-inflammatoire du traitement doit se traduire par une diminution de l'érythème et par une diminution de la gingivite.

Cliniquement, l'érythème se traduit par un aspect érythémateux (couleur rouge) de la gencive et par un grade élevé de la gingivite. Un retour à l'état normal de la gencive, une diminution de l'érythème, du stade de la gingivite et de l'étendue de l'atteinte, constituent les preuves cliniques d'une activité anti-inflammatoire.

Les critères secondaires sont l'appréciation de la douleur spontanée et à la mastication, du saignement (intensité et topographie).

### Exploitation des critères :

Les trois critères principaux témoignant de l'importance de l'activité inflammatoire, "gingivite", "couleur de la gencive" et "topographie de l'atteinte", reçoivent un coefficient 2 au niveau de l'expression des résultats.

Les indices "douleurs spontanées", "douleur à la mastication" et "saignement papillaire" (intensité et topographie), recevront un coefficient 1 au niveau de l'expression des résultats.
- Pour l'évaluation de la gingivite, la cotation est la suivante :
   - (0): si le grade est 0 : gencive normale, pas d'inflammation, pas de changement de couleur, pas de saignement ;
   - (2): si le grade est 1 : gingivite peu étendue, légère d'inflammation, légère altération de la surface gingivale, pas de saignement ;
   - (4): si le grade est 2 : gingivite d'extension modérée, inflammation modérée, érythème, oedème, saignement du sondage ou après pression ;
   - (6): si le grade est 3 : gingivite très étendue, inflammation sévère, érythème important, oedème, tendance au saignement spontané, ulcération.
- Pour l'évaluation de la couleur de la gencive, la cotation est la suivante :
   - (0): normale
   - (2): érythème léger
   - (4): érythème modéré
   - (6): érythème sévère
- L'évaluation topographique de la couleur de la gencive :
   Six secteurs sont définis : DS, AS, GS, DI, AI, et GI (A, S, I, D et G signifient respectivement Avant, Supérieur, Inférieur, Droite et Gauche).
   - (0): aucun secteur atteint
   - (2): un secteur atteint
   - (4): deux secteurs atteints
   - (6): trois secteurs atteints
   - (8): plus de trois secteurs atteints
- Pour l'évaluation du saignement papillaire (Engelberger et Coll., 1993), la cotation est la suivante :
   - (0): si le grade est 0 : pas de saignement 10 à 30 secondes après sondage
   - (2): si le grade est 1 : saignement léger (fine traînée de sang)
   - (4): si le grade est 2 : saignement modéré
   - (6): si le grade est 3 : saignement intense
- L'évaluation topographique du saignement papillaire :
   - (0): aucun secteur atteint
   - (1): un secteur atteint
   - (2): deux secteurs atteints
   - (3): trois secteurs atteints
   - (4): plus de trois secteurs atteints
- Les modifications des critères "douleur spontanée et à la mastication" sont évaluées à l'aide d'une échelle visuelle à J₀ et J₂₈:
   - (0): si pas de douleur ou douleur faible (mesure entre 0 et 2,5 cm sur l'échelle visuelle)
   - (1): si douleur modérée (mesure entre 2,5 et 5 cm sur l'échelle visuelle)
   - (2): si douleur importante (mesure entre 5 et 7,5 cm sur l'échelle visuelle)
   - (3): si douleur sévère (mesure entre 7,5 et 10 cm sur l'échelle visuelle)
- Un indice global est ensuite calculé en sommant les différents indices.
   . le retour à l'état normal reçoit l'indice 0.
   . la gravité maximale reçoit l'indice 33.

Les résultats sont rassemblés dans le tableau 2 ci-dessous.

**Tableau 2 :**

| | Jour J₀ | Jour J28 |
|---|---|---|
| Evaluation de la gingivite | 3,50 ± 1 | 1,0 ± 1,2 (p<0,05) |
| Evaluation de la couleur de la gencive (érythème) | 4,00 ± 1,4 | 1,1 ± 1,45 (p<0.05) |
| Topographie de l'érythème gingival | 4,80 ± 1,7 | 1,6 ± 11,8 (p<0,05) |
| Evaluation du saignement papillaire | 0,85 ± 0,8 | 0,1 ± 0,43 (p<0,05) |
| Topographie du saignement papillaire | 1,20 ± 1,12 | 0,1 ± 0,43 (p<0,05) |
| Evaluation de la douleur spontanée | 1,35 ± 1,2 | 0 (p<0,05) |
| Evaluation de la douleur à la mastication | 1,10 ± 1,17 | 0 (p<0,05) |
| Score global | 16,80 ± 4,8 | 3,9 ± 4,6 (p<0,05) |

Cette étude sur 20 patients atteints de gingivite tartrique, a permis de mettre en évidence l'activité anti-inflammatoire d'un extrait de ginkgo tel que défini précédemment en association avec des céramides végétales.

Cette activité anti-inflammatoire se traduit cliniquement de façon significative, par une disparition de la gingivite et de l'érythème gingival dans 11 cas sur 20 et par une amélioration dans 8 autres cas.

Il a été observé une amélioration significative de l'indice de saignement papillaire et une disparition complète, dans tous les cas, de la douleur gingivale (spontanée et à la mastication).

## Revendications

1. Utilisation d'un extrait flavonoïdique de feuilles de ginkgo biloba substantiellement dépourvu de terpènes pour la préparation d'une composition pharmaceutique destinée au traitement des affections de la sphère buccodentaire.

2. Utilisation selon la revendication 1, **caractérisée en ce que** le dit extrait comprend de 28 à 35 % d'hétérosides flavonoïdiques, et de préférence 28 à 32 %.

3. Utilisation selon l'une des revendications 1 à 2, **caractérisée en ce que** le dit extrait comprend au maximum 1 % de terpènes, de préférence au maximum 0,5 % de terpènes.

4. Composition pharmaceutique destinée notamment au traitement de la sphère bucco-dentaire et contenant comme principe actif un extrait flavonoïdique de feuilles de ginkgo biloba substantiellement dépourvu de terpènes ainsi que des excipients.

5. Composition cosmétique destinée notamment à la sphère buccodentaire et contenant un extrait flavonoïdique de feuilles de ginkgo biloba substantiellement dépourvu de terpènes.

6. Composition selon l'une des revendications 4 à 5, **caractérisée en ce que** le dit extrait comprend de 28 à 35 % d'hétérosides flavonoïdiques, et de préférence 28 à 32 %.

7. Composition selon l'une des revendications 4 à 6, **caractérisée en ce que** le dit extrait comprend au maximum 1 % de terpènes, et de préférence au maximum 0,5 % de terpènes.

8. Composition selon l'une des revendications 4 à 7, **caractérisée en ce qu'**elle contient de 0,05 à 0,6 % du dit extrait.

9. Composition selon l'une des revendications 4 à 8, **caractérisée en ce que** le dit extrait est associé à des céramides.

10. Composition selon la revendication 9, **caractérisée en ce qu'**elle contient de 0,05 à 0,3 % de céramides.

11. Composition selon la revendication 10, sous forme de gel ou de chewing-gum.
